(19) Europäisches Patentamt
European Patent Office
Office européen des brevets

(11) **EP 2 946 741 A1**

(12) **EUROPEAN PATENT APPLICATION**
published in accordance with Art. 153(4) EPC

(43) Date of publication:
**25.11.2015 Bulletin 2015/48**

(51) Int Cl.:
*A61B 19/00* (2006.01)       *A61B 1/313* (2006.01)

(21) Application number: **14740207.7**

(22) Date of filing: **15.01.2014**

(86) International application number:
**PCT/KR2014/000426**

(87) International publication number:
**WO 2014/112782 (24.07.2014 Gazette 2014/30)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **18.01.2013   KR 20130005807**

(71) Applicants:
• **Koh Young Technology Inc.**
  **Seoul 153-706 (KR)**
• **Kyungpook National University Industry-
  Academic
  Cooperation Foundation
  Daegu 702-701 (KR)**

(72) Inventors:
• **HONG, Jong-Kyu**
  **Gwangju-si**
  **Gyeonggi-do 464-892 (KR)**
• **LEE, Hyun-Ki**
  **Daegu 706-760 (KR)**
• **CHUNG, Jae-Heon**
  **Gwangmyeong-si**
  **Gyeonggi-do 423-733 (KR)**
• **KIM, Min-Young**
  **Daegu 706-819 (KR)**

(74) Representative: **Zardi, Marco**
  **M. Zardi & Co. SA**
  **Via Pioda 6**
  **6900 Lugano (CH)**

(54) **TRACKING SYSTEM AND TRACKING METHOD USING SAME**

(57)     A tracking system and method using the same is disclosed which is capable of minimizing a restriction of surgical space by achieving a lightweight of the system as well as a reduction of a manufacturing cost through calculating a three-dimensional coordinates for each maker using one image forming unit. In the tracking system and method using the same, lights emitted from each marker are passed through a lens array unit which includes at least a pair of lens, images of the markers corresponding to the number of the lenses of lens array units are formed on an image forming for each marker, and therefore, it is possible to calculate a spatial position and a direction of the markers attached on the target by using only one image forming unit through a trigonometry. Also, since the system is not affected by lens array method and magnification, there is an effect of reducing a manufacturing cost of the tracking system with small and light-weight, and relatively low restriction of surgical space comparing with conventional tracking system.

FIG. 1

EP 2 946 741 A1

**Description**

TECHNICAL FIELD

Exemplary embodiments of the present invention relate to a tracking system and tracking method using the same. More particularly, exemplary embodiments of the present invention relate to a tracking system and tracking method using the same for surgery capable of detecting spatial and direction information of a target by tracking coordinates of markers attached on the target such as a surgical instrument of an affected area.

BACKGROUND

Recently, a robot surgery have been studied and introduced to reduce pain of patients and to recover faster in an endoscopic surgery or an otolaryngology surgery (ENT surgery).

In such a robot surgery, in order to minimize risk of the surgery and to operate the surgery more precisely, a navigation system is used to navigate to an exact lesion of a patient by tracking and detecting a spatial position and a direction of a target such as lesion portion or surgical instrument.

The navigation system described above includes a tracking system which is capable of tracking and detecting a spatial position and a direction of a target such as lesion or surgical instrument.

The tracking system described above includes a plurality of markers attached on a lesion or a surgical instrument, a first and second image forming units forming images of lights emitted from the markers, and a processor calculating three-dimensional coordinates of the markers which are connected to the first and second image forming units and calculating a spatial position and a direction of the target by comparing pre-stored information of straight lines which connect the markers adjacent to each other and, angle information which are formed by a pair of straight lines adjacent to each other with the three-dimensional coordinates of the markers.

Herein, in order to calculate the three-dimensional coordinates of the markers, conventionally, two detectors are required to calculate the three-dimensional coordinates of each marker through a processor, a trigonometry is used in an assumption that a coordinate of marker which is emitted from one marker and forming image on a first image forming unit and a coordinate of marker which is emitted from one marker and forming image on a second image forming unit are identical.

Conventional tracking system requires two image forming units to form images of lights which are emitted from each marker in different position to each other, manufacturing cost increases as well as a whole size also increases, therefore, a restriction of a surgical space is generated.

SUMMARY

Therefore, the technical problem of the present invention is to provide a tracking system and method using the same capable of reducing manufacturing cost as well as minimizing restriction of a surgical space by achieving compact of the system through calculating three-dimensional coordinates of each of markers by using only one image forming unit.

In one embodiment of the present invention, a tracking system includes at least three markers which are attached on a target and emit lights or reflect lights emitted from a light source, a lens array unit in which at least two lenses are arranged at an interval to pass the lights emitted from the markers, an image forming unit which receives the lights that are emitted from the markers and have passed the lens array and forms images corresponding to the number of the lenses of the lens array unit, and a processor which calculates a three-dimensional coordinates of each marker using the images corresponding to the number of the lenses of lens array unit, compares the three-dimensional coordinates of the markers with pre-stored geometric information of markers which are adjacent to each other, and calculates a spatial position and direction of the target.

In one embodiment, the markers may be self-luminous active markers.

Meanwhile, the tracking system may further include at least one light source emitting light from the lens array unit to the markers, in this case, the markers may be passive markers which reflect light from the light source to the lens array unit.

In one embodiment, the image forming unit may be a camera which receives lights that are emitted from the markers and have passed each lens of the lens array unit, and forms at least two images corresponding to the number of the lenses of the lens array unit for each marker.

In one embodiment, geometric information of the markers may be length information connecting markers adjacent to each other and angle information formed by a pair of straight lines adjacent to each other.

In one embodiment of the present invention, a tracking method includes emitting lights by at least three markers attached on a target, forming images corresponding to the number of the lenses of lens array unit, in which the lens array unit includes at least two lenses and lights emitted from the markers pass through them, calculating three-dimensional coordinates for each marker by using the images that are formed on the image forming unit corresponding to the number of the lenses of lens array unit for each marker through the processor and, calculating a spatial position and a direction of the target by comparing the three-dimensional coordinates of the each marker with pre-stored geometric information of markers adjacent to each other.

In some embodiment, geometric information of the markers may be length information connecting markers adjacent to each other, and angle information formed by a pair of straight lines adjacent to each other.

Meanwhile, the process of calculating three-dimensional coordinates of the marker may include calculating two-dimensional coordinates of the images corresponding to the number of the lenses of lens array unit formed on the image forming unit for each marker through the processor and, calculating three-dimensional coordinates of the markers by using the two-dimensional coordinates of the images corresponding to the number of the lenses of lens array unit for each marker.

In some embodiment, the process of emitting lights by the markers, markers may self-emit lights to the lens array unit.

Alternatively, the process of emitting lights by the markers, at least a light source is used to emit light, and the light is reflected from the lens array unit through the markers.

Meanwhile, a spatial position and a direction of the light source are pre-stored in the processor.

## ADVANTAGEOUS EFFECTS

Thus, according to an embodiment of the present invention, in a tracking system and tracking method using the same, lights emitted from each marker pass through a lens array unit which includes at least a pair of lens, images corresponding to the number of the lenses of lens array units are formed on an image forming for each marker, and therefore, it is possible to calculate a spatial position and a direction of the markers attached on the target by using only one image forming unit through a trigonometry.

Also, since the system is not affected by lens array method and magnification, there is an effect of reducing manufacturing cost of the tracking system, making small and lightweight, and relatively low restriction of a surgical space compared with the conventional tracking system.

## BRIEF DESCRIPTION OF THE DRAWINGS

FIG.1 is a schematic diagram of a tracking system according to an embodiment of the present invention.

FIG. 2 is an example diagram of markers attached on a target.

FIG. 3 is an example diagram explaining a change of image forming position of a marker when a position of the marker is changed in a same optical path as a lens.

FIG. 4 is a block diagram explaining a tracking method according to an embodiment of the present invention.

FIG. 5 is a block diagram explaining a method of calculating three-dimensional coordinates.

FIG. 6 is an example diagram of an image sensor of the image forming unit in which a coordinate of a first marker and a coordinate of a second marker are virtually divided.

FIG. 7 is a diagram explaining a relationship between two-dimensional coordinates and three-dimensional coordinates of a real marker.

## DETAILED DESCRIPTION

The present invention is described more fully hereinafter with reference to the accompanying drawings, in which example embodiments of the present invention are shown. The present invention may, however, be embodied in many different forms and should not be construed as limited to the example embodiments set forth herein. Rather, these example embodiments are provided so that this disclosure will be thorough and complete, and will fully convey the scope of the present invention to those skilled in the art. In the drawings, the sizes and relative sizes of layers and regions may be exaggerated for clarity.

It will be understood that, although the terms first, second, third, etc. may be used herein to describe various elements, components, regions, layers and/or sections, these elements, components, and/or sections should not be limited by these terms. These terms are only used to distinguish one element, component, region, layer or section from another region, layer or section. Thus, a first element, component, or section discussed below could be termed a second element, component, or section without departing from the teachings of the present invention.

The terminology used herein is for the purpose of describing particular example embodiments only and is not intended to be limiting of the present invention. As used herein, the singular forms "a," "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

Hereinafter, with reference to the drawings, preferred embodiments of the present invention will be described in detail.

In a tracking system and method using the same according to an embodiment of the present invention, at least three markers are attached on a patient or a surgical instrument, three-dimensional coordinates of the markers are calculated, the three-dimensional coordinates of the markers are compared with pre-stored geometric information of markers adjacent to each other through a processor, and therefore, it is capable of calculating a spatial position and a direction of a target such as a lesion or surgical instrument. The detailed description is explained referencing figures.

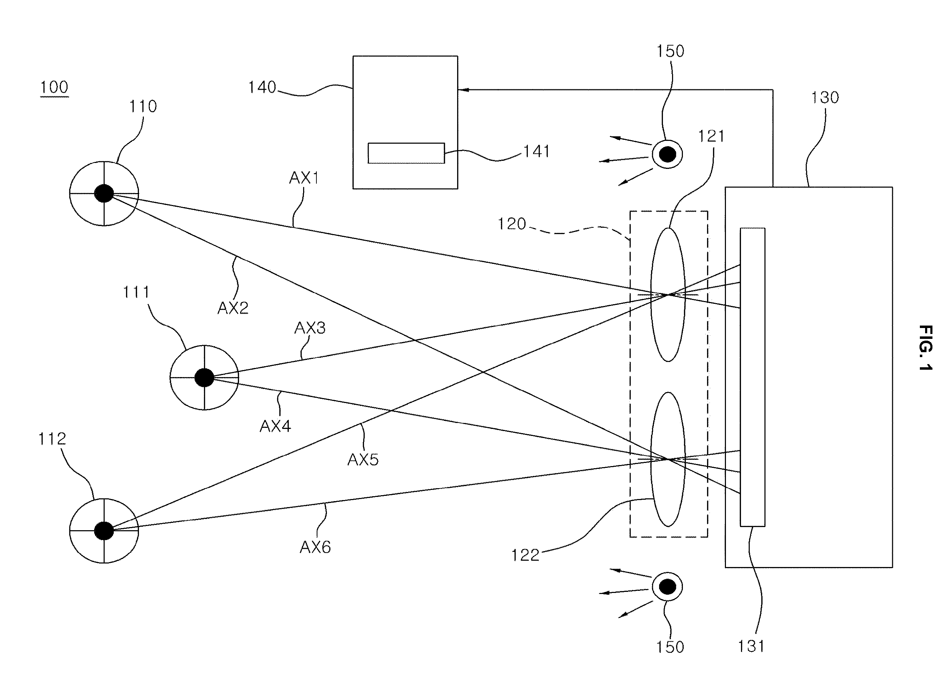

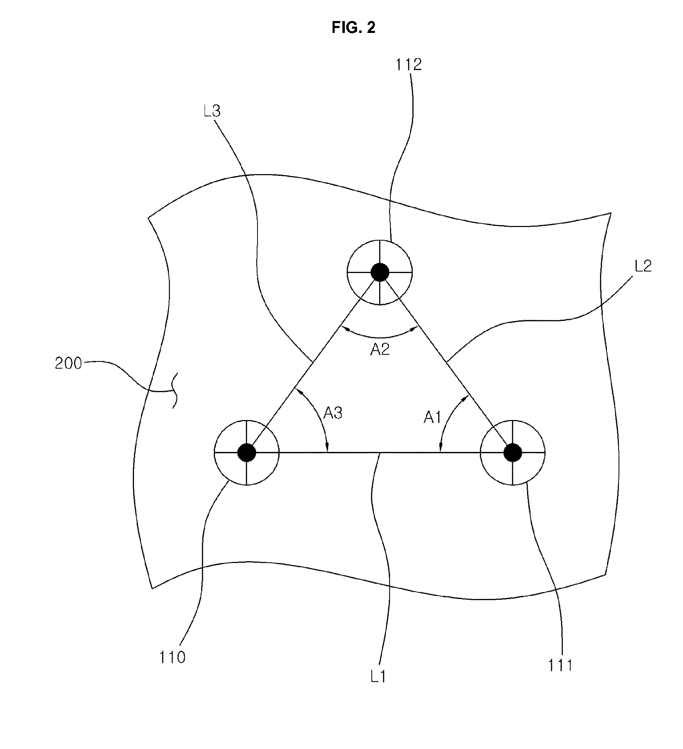

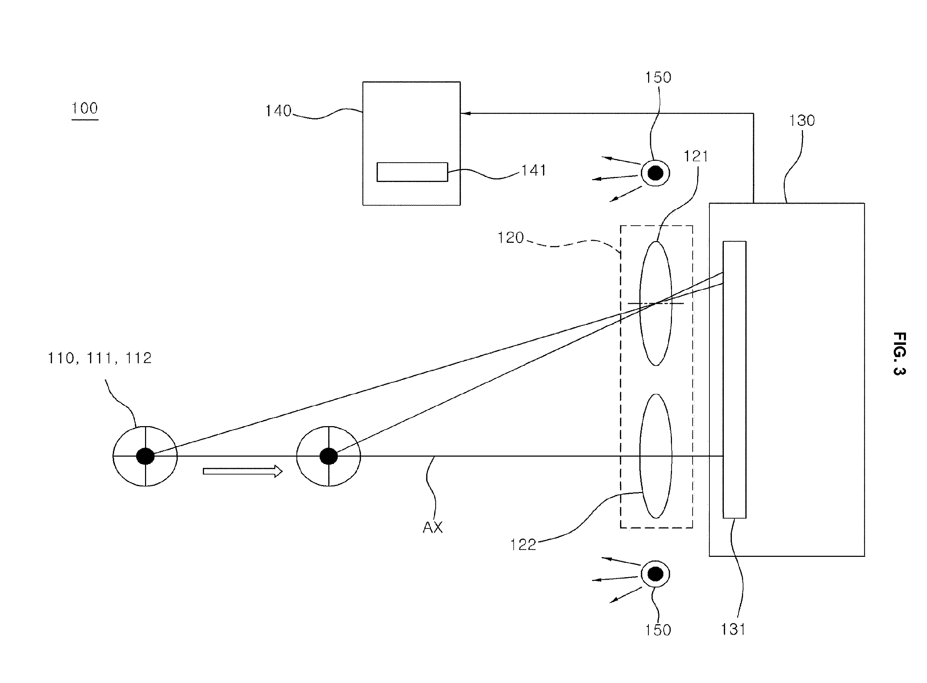

**FIG.1** is a schematic diagram of a tracking system according to an embodiment of the present invention, **FIG. 2** is an example diagram of markers attached on a target, and **FIG. 3** is an example diagram explaining a change of image forming position when a position of the marker is changed in a same optical path as a lens.

Referring to **FIGS. 1** to **3**, a tracking system according to an embodiment of the present invention includes at least three markers 110, 111 and 112, a lens array unit 120, an image forming unit 130, and a processor 140, herein, the lens array unit 120 may be included and installed on the image forming unit 130.

The at least three markers 110, 111 and 112 are attached on the target 200 such as a lesion or a surgical instrument. Herein, the at least three markers 110, 111 and 112 are separated at an interval each other, the adjacent markers 110, 111 and 112 are arranged on the target 200 such as the lesion or the surgical instrument to form specific angles A1, A2 and A3 formed by a pair of straight lines L1, L2 and L3 in which the adjacent markers are virtually connected to each other.

Herein, geometric information between the markers 110, 111 and 112 adjacent to each other, in other words, length information of straight lines L1, L2 and L3 which connect the markers 110, 111 and 112 adjacent to each other and angle information A1, A2 and A3 formed by a pair of straight lines connecting the adjacent markers, are stored in a memory 141 of the processor 140.

For example, the markers 110, 111 and 112 may be attached on the target 200 such as a lesion and a surgical instrument in a triangle shape, length information of straight lines L1, L2 and L3 forming sides of the triangle in which the markers are used as vertices and, angle information A1, A2 and A3 formed by a pair of straight lines connecting the adjacent markers may be pre-stored in the memory 141 included in the processor 140.

Meanwhile, the markers 110, 111 and 112 may be active markers which self-emit lights. As described above, a light source is not needed when active markers are used as the markers 110, 111 and 112.

Alternatively, the markers 110, 111 and 112 may be passive markers which reflect light emitted from at least one light source 150.

As described above, at least one light source 150 may be arranged close to the lens array unit 120 when passive markers are used for the markers 110, 111 and 112. For example, a pair of light source 150 may be arranged on both sides of the lens array unit 120. Herein, a spatial position and a direction of the light source 150 are pre-stored the memory 141 which is integrated in the processor 140.

The lens array unit 120 is arranged on a front side of the image forming unit 130. At least a pair of lenses 121 and 122 are arranged and formed on such a lens array unit 120 at an interval to pass the lights emitted from the markers 110, 111 and 112. For example, the first lens 121 and the second lens 122 may be arranged and formed on the lens array unit 120 at an interval. Although the first and second lenses 121 and 122 are arranged on the lens array unit 120 at an interval as shown in the figure, however, more than three lenses may be formed at an interval on the lens array unit 120.

The image forming unit 130 receives lights which are emitted from the markers 110, 111 and 112 and have passed each lens of the lens array unit 120, and forms images corresponding to the number of the lenses of lens array unit 120 for each marker.

In more detail, when the first and second lenses 121 and 122 are arranged on the lens array unit 120 at an interval, the image forming unit 130 receives the lights that are emitted from the markers 110, 111 and 112 and having passed the first and second lenses 121 and 122, and forms pair of images for each marker.

For example, the image forming unit 130, which receives lights that are emitted from the markers 110, 111 and 112 and have passed each lens of the lens array unit 120 and forms number of images corresponding to the number of the lenses of lens array unit 120 for each marker, may be a camera in which an image sensor 131 is integrated in it.

The processor 140 calculates three-dimensional coordinates of the markers 110, 111 and 112 by using the images corresponding to the number of the lenses of the lens array unit for each, and calculates a spatial position and a direction of the target 200 such as a lesion or surgical instrument by comparing the three-dimensional coordinates of the markers 110, 111 and 112 with the pre-stored geometric information of the adjacent markers 110, 111 and 112.

Herein, the memory 141 is integrated in the processor 140. Meanwhile, geometric information between the markers adjacent to each other, in other words, length information of straight lines L1, L2 and L3 which connect the marker adjacent to each other and angle information A1, A2 and A3 formed by the pair of straight lines connecting the markers 110, 111 and 112 adjacent to each other, may be pre-stored the memory 141 integrated in the processor 140.

Additionally, when the markers 110, 111 and 112 are passive markers, a spatial position and a direction of the pair of light sources 150 may be pre-stored in the memory 141 integrated in the processor 140.

As described above, in the tracking system 100 according to an embodiment of the present invention, the lens array unit 120 using at least a pair of lenses 121 and 122 are arranged at an interval is used to pass lights emitted from the markers 110, 111 and 112, the lights emitted from the markers 110, 111 and 112 pass through at least a pair of lenses 121 and 122, at least a pair of images are formed on the image forming unit 120 for each marker, and therefore, there is an advantage of calculating a three-dimensional coordinates for each marker by using only one image forming unit 130.

For example, as shown in **FIG. 3**, when a position of the markers 110, 111 and 112 are changed in the same optical axis AX, a position of the image sensor 133, in which an image of the second lens 122 is formed, is not changed, however, a position of the image sensor 133, in which an image of the first lens 121 is formed, is changed, therefore, it is possible to calculate a three-dimensional coordinates for each marker by using only one image forming unit 130 through a trigonometry.

Referring to **FIGS. 1** to **7**, a tracking process of a spatial position and a direction of a target using a tracking system according to an embodiment of the present invention is described below.

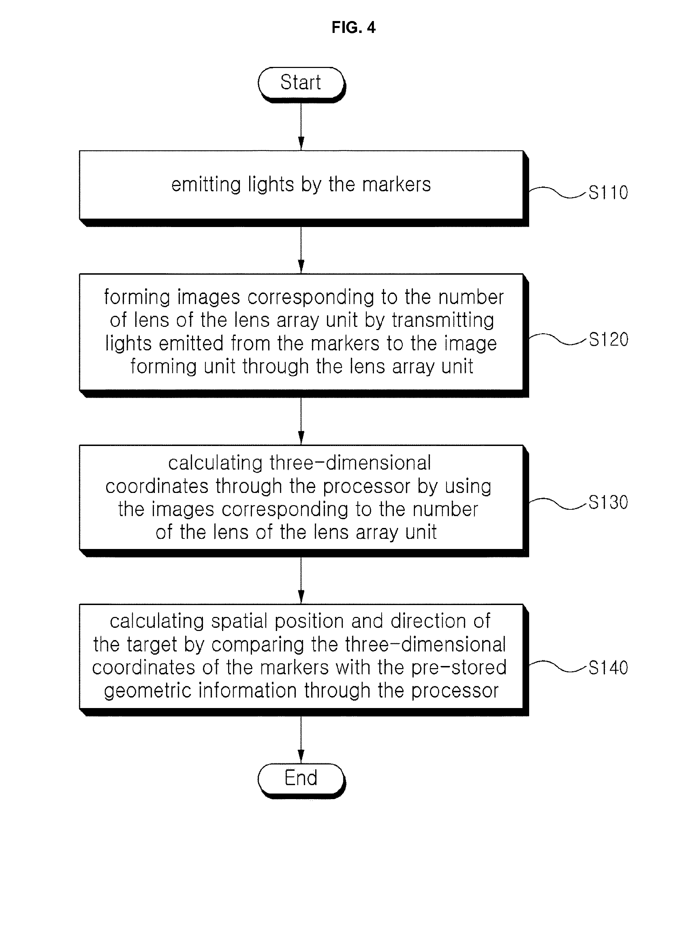

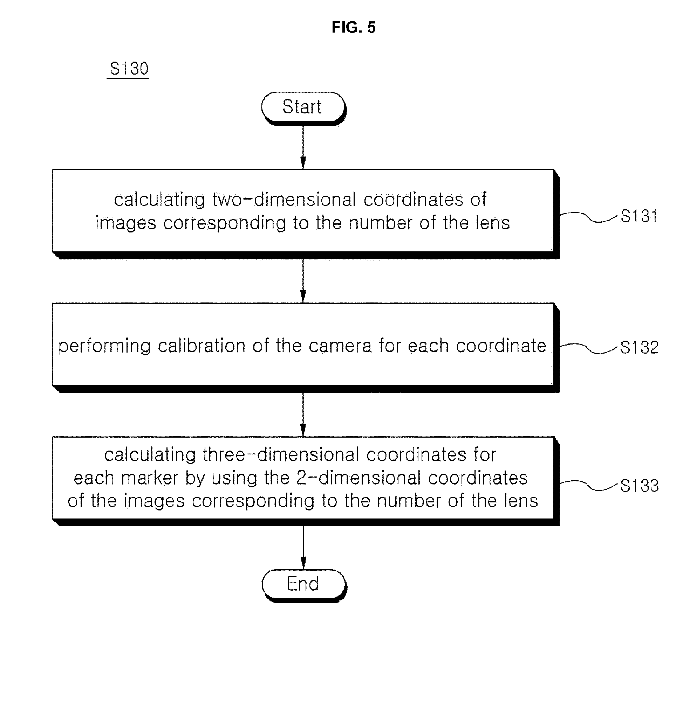

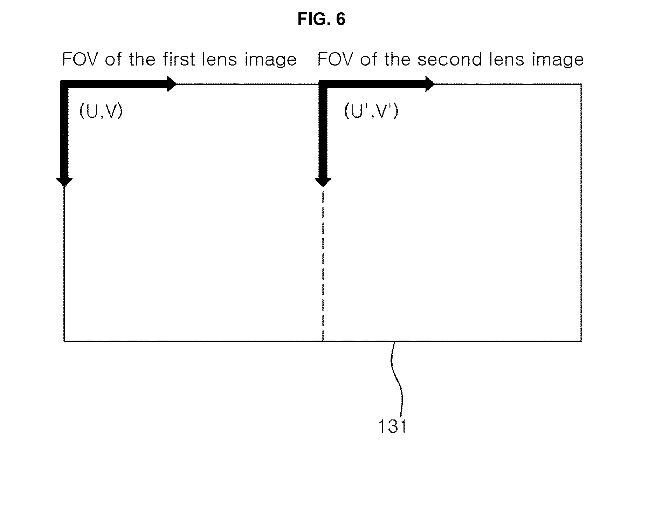

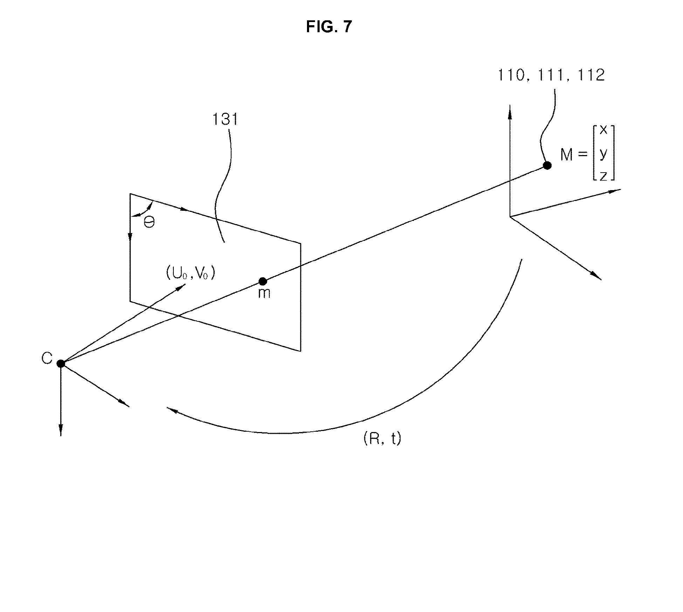

**FIG. 4** is a block diagram explaining a tracking method according to an embodiment of the present invention, **FIG. 5** is a block diagram explaining a method of calculating three-dimensional coordinates, **FIG. 6** is an example diagram of an image sensor of the image forming unit in which a coordinate of a first marker and coordinate of a second marker are virtually divided, and **FIG. 7** is a diagram explaining a relationship between two-dimensional coordinates and three-dimensional coordinates of a real marker.

Referring to **FIGS. 1** to **7**, in order to track a spatial position and direction of a target 200 using a tracking system according to an embodiment of the present invention, first, at least three markers 110, 111 and 112 attached on the target 200 are activated making the markers 110, 111 and 112 to emit light, or, at least one light source 150 is activated to irradiated light toward the markers 110, 111 and 112 attached on the target 200 such that the lights are reflected by the markers 110, 111 and 112 (S110).

In more detail, when at least three active (self-luminous) markers 110, 111 and 112 are attached on the target 200, the markers 110, 111 and 112 are activated to emit lights. Alternatively, when at least three passive (non-self-luminous) markers 110, 111 and 112 are attached on the target 200, at least one light source 150 is activated to irradiate light toward the passive markers 110, 111 and 112 attached on the target 200 such that the lights are reflected by the passive markers 110, 111 and 112.

The lights emitted from the at least three markers 110, 111 and 112 pass through each lenses 121 and 122 of the lens array unit 120, and images corresponding to the number of the lenses of the lens array unit 120 are formed on the image forming unit 130 (S120).

For example, as shown in **FIG. 1**, when a lens array unit 120 including a pair of first and second lenses 121 and 122 and arranged at an interval is used, light emitted from the first marker 110 passes each of the first lens 121 and second lens 122 through a first optical axis AX1 and second optical axis AX2 and an image of the first marker is formed on the image forming unit 130, light emitted from the second marker 111 passes each of the first lens 121 and second lens 122 through a third optical axis AX3 and fourth optical axis AX4 and an image of the second marker is formed on the image forming unit 130, and light emitted from the third marker 112 passes each of the first lens 121 and second lens 122 through a fifth optical axis AX5 and sixth optical axis AX6 and an image of the third marker is formed on the image forming unit 130.

In other words, a pair of images are formed on the image forming unit 130 for each marker 110, 111 and 112 by using the lens array unit 120 including a pair of first and second lenses 121 and 122 arranged at an interval.

When images corresponding to the number of the lenses of lens array unit are formed on the image forming unit 130 for each marker 110, 111 and 112, three-dimensional coordinates of each marker 110, 111 and 112 are calculated by using the images corresponding to the number of the lenses of lens array unit are formed on the image forming unit 130 for each marker 110, 111 and 112 (S130).

**FIG. 5** shows a detailed process of calculating three-dimensional coordinates for each marker 110, 111 and 112. For the convenience of explanation, it will be described as an example in the case of using the lens array unit 120 in which the first and second lenses 121 and 122 are arranged at an interval.

In order to calculate three-dimensinal coordinates of the markers 110, 111 and 112, first, two-dimensional coordinates of a pair of images formed on the image forming unit 130 for each marker 110, 111 and 112 are calculated through the processor 140 (S131).

Herein, after calculating two-dimensional coordinates of the markers 110, 111 and 112, a camera calibration of the image forming unit 130 is processed for each coordinates (FOV(field of view) of image of the first lens and FOV of image of the second lens) (S 132).

As described above, after processing a camera calibration, three-dimensional coordinates of each of the markers 110, 111 and 112 are calculated by using the two-dimensional coordinates of the pair of images formed for each marker 110, 111 and 112 (S133).

Referring to **FIGS. 6** and **7**, a detailed process of calculating three-dimensional coordinates of each of the markers 110, 111 and 112 is described in below.

As shown in **FIG. 6**, one side of the image sensor 133 is virtually divided in a FOV (field of view) of the image of the first lens and another side of the image sensor is virtually divided in a FOV (field of view) of the image of the second lens, two-dimensional coordinates of the direct image of the image sensor 133 are represented by a coordinate system (U,V), and two-dimensional coordinates of the reflected image of the image sensor 133 are represented by a coordinate system (U',V'). Referring to **FIG. 7**, a relationship between the 2-dimensional coordinates of the markers 110, 111 and 112 in real space and the three-dimensional coordinates of the markers 110, 111 and 112 in real space may be represented in a formula below.

[Formula 1]

$$s \begin{bmatrix} u \\ v \\ 1 \end{bmatrix} = \begin{bmatrix} \alpha & \gamma & u_0 \\ 0 & \beta & v_0 \\ 0 & 0 & 1 \end{bmatrix} \underbrace{\begin{bmatrix} r_1 & r_2 & r_3 & t \end{bmatrix}}_{[R,\,t]} \begin{bmatrix} x \\ y \\ z \\ 1 \end{bmatrix}$$

$$\underbrace{\phantom{\begin{bmatrix} u \\ v \\ 1 \end{bmatrix}}}_{\tilde{m}} \quad \underbrace{\phantom{\begin{bmatrix} \alpha \\ 0 \\ 0 \end{bmatrix}}}_{A} \qquad \underbrace{\phantom{\begin{bmatrix} x \\ y \\ z \\ 1 \end{bmatrix}}}_{\tilde{M}}$$

Herein, m is two dimensional coordinates of the markers in the image, M is three-dimensional coordinates of the markers in real space, and A(R, t) is a matrix of the camera.

In order to explain more briefly, when three-dimensional coordinates of real markers 110, 111 and 112 are represented in X, a relational formula between three-dimensional coordinates of real markers 110, 111 and 112 and coordinates of direct image $x_L$, and a relational formula between three-dimensional coordinates of real markers 110, 111 and 112 and coordinates of reflected image $x_R$ are represented in below.

[Formula 2]

$$x_L = P_1 X$$

$$x_R = P_2 X$$

Herein, $P_1$ is a camera matrix of the direct image, and $P_2$ is a camera matrix of the reflected image.

And, relation formulas of the direct image and reflected image of each of the markers 110, 111 and 112, $x_L = P_1 X$, $x_R = P_2 X$, may be represented in a linear equation AX= 0, and the equation may be represented in Formula 3.

[Formula 3]

$$x(P^{3T}X) - (P^{1T}X) = 0$$

$$y(P^{3T}X) - (P^{2T}X) = 0$$

$$x(P^{2T}X) - y(P^{1T}X) = 0$$

Herein, P$^{jT}$ is a row vector of the matrix P.

The formula 3 may be represented in Formula 4.

[Formula 4]

$$\begin{bmatrix} x_L P_1^{3T} - P_1^{1T} \\ y_L P_1^{3T} - P_1^{2T} \\ x_R P_2^{3T} - P_2^{1T} \\ y_R P_2^{3T} - P_2^{2T} \end{bmatrix} \begin{bmatrix} X \\ Y \\ Z \\ w \end{bmatrix} = [0]$$

Herein, W may be a scale factor.

Three-dimensional coordinates of the markers 110, 111 and 112 are obtained by calculating X, Y, and Z through solving the linear equation represented in formula 4.

As described in **FIGS. 4** and **5**, after calculating three-dimensional coordinates of each marker 110, 111 and 112 through the processor 140, three-dimensional coordinates of the markers 110, 111 and 112 in real space are compared with pre-stored geometric information of the markers adjacent to each other through the processor 140, and a spatial position and a direction of the markers 110, 111 and 112 attached on the target 200 are calculated (S140).

Herein, as described above, geometric information between the adjacent markers 110, 111 and 112 may be length information of straight lines L1, L2 and L3 which connect the marker adjacent to each other and angle information A1, A2 and A3 formed by the pair of straight lines connecting the markers 110, 111 and 112 adjacent to each other.

In other words, a spatial position and a direction of the target in which the markers 110, 111 and 112 are attached are calculated by comparing the three-dimensional coordinates of the markers 110, 111 and 112 in real space with pre-stored length information of straight lines L1, L2 and L3 which connect the marker adjacent to each other and pre-stored angle information A1, A2 and A3 formed by the pair of straight lines connecting the markers 110, 111 and 112 adjacent to each other.

As described above, a tracking system and method using the same according to an embodiment of the present invention, the lights emitted from each marker 110, 111 and 112 pass to an image forming unit 130 including at least a pair of lenses, and images corresponding to the number of the lenses of the lens array unit 130 for each marker are formed on the image forming unit 130.

In other words, when a lens array unit 120 including a pair of first and second lenses 121 and 122 is used, two images are formed on the image forming unit 130 through two optical axis for each marker, and therefore, three-dimensional coordinates of the markers are calculated by using only one image forming unit 130 through a trigonometry.

Therefore, a tracking system and method using the same according to an embodiment of the present invention, one image forming unit 130 is used to calculate a spatial position and a direction of the markers 110, 111 and 120 attached on the target 200.

Therefore, there is an effect of reducing a manufacturing cost of the tracking system, making small and lightweight, and relatively low restriction of surgical space compared with the conventional tracking system.

It will be apparent to those skilled in the art that various modifications and variation can be made in the present invention without departing from the scope of the invention. Thus, it is intended that the present invention cover the modifications and variations of this invention provided they come within the scope of the appended claims and their equivalents.

## Claims

1. A tracking system comprising:

    at least three markers attached on a target to emit lights;
    a lens array unit including at least two lenses arranged at an interval to pass the lights emitted from the markers;
    an image forming unit receiving the lights that are emitted from the markers and have passed through each lens of the lens array unit, and forming images corresponding to the number of the lenses of the lens array unit for each marker; and
    a processor calculating three-dimensional coordinates of the markers by using the images of the markers that are formed on the image forming unit corresponding to the number of the lenses of the lens array unit for each marker, and calculating a spatial position and a direction of the target by comparing the three-dimensional coordinates of the markers with pre-stored geometric information between the markers adjacent to each other.

2. The tracking system of claim 1, wherein the markers are self-luminous active markers.

3. The tracking system of claim 1, further comprising at least one light source emitting light from the lens array unit toward the markers, wherein the markers are passive markers reflecting the emitted light from the light source toward the lens array unit.

4. The tracking system of claim 1, wherein the image forming unit is a camera forming at least two images corresponding to the number of the lens of lens array unit for each marker by receiving lights emitted from the markers and having passed through each lens of the lens array unit.

5. The tracking system of claim 1, wherein the geometric information between the markers comprises length information of straight lines which connect the markers adjacent to each other, and angle information which is formed by a pair of straight lines adjacent to each other.

6. A tracking method comprising:

emitting lights by at least three markers attached on a target;
forming images corresponding to the number of the lenses of a lens array unit formed on an image forming unit, wherein the images are formed by the lights emitted from the markers and having passed through the at least two lenses of the lens array unit;
calculating three-dimensional coordinates for each marker through a processor by using the images of the markers formed on the image forming unit corresponding to the number of the lenses of the lens array unit; and calculating a spatial position and a direction of the target by comparing the three-dimensional coordinates of each marker with geometric information between the markers adjacent to each other, wherein the geometric information is pre-stored in a processor.

7. The tracking method of claim 6, wherein the geometric information comprises length information of straight lines which connect the markers adjacent to each other and, angle information which are formed by a pair of straight lines adjacent to each other.

8. The tracking method of claim 6, wherein calculating the three-dimensional coordinates of the markers comprises:

calculating two-dimensional coordinates of the images of the markers corresponding to the number of the lenses of the lens array unit formed on the image forming unit for each marker through the processor; and calculating the three-dimensional coordinates of the markers through the processor by using the two-dimensional coordinates of the images of the markers corresponding to the number of the lenses of the lens array unit for each marker.

9. The tracking method of claim 6, wherein in emitting lights by the markers, the markers self-emit light toward the lens array unit.

10. The tracking method of claim 6, wherein in emitting lights by the markers, light emitted from at least one light source is reflected to the lens array unit through the markers.

11. The tracking method of claim 10, wherein a spatial position and a direction of the light source are pre-stored in the processor.

## FIG. 1

**FIG. 2**

**FIG. 3**

**FIG. 4**

```
         ┌──────────┐
         │  Start   │
         └────┬─────┘
              │
              ▼
┌────────────────────────────────────┐
│                                    │
│   emitting lights by the markers   │          S110
│                                    │
└────────────────┬───────────────────┘
                 │
                 ▼
┌────────────────────────────────────┐
│  forming images corresponding to   │
│  the number of lens of the lens    │
│  array unit by transmitting        │          S120
│  lights emitted from the markers   │
│  to the image forming unit through │
│  the lens array unit               │
└────────────────┬───────────────────┘
                 │
                 ▼
┌────────────────────────────────────┐
│     calculating three-dimensional  │
│  coordinates through the processor │
│  by using the images corresponding │          S130
│  to the number of the lens of the  │
│  lens array unit                   │
└────────────────┬───────────────────┘
                 │
                 ▼
┌────────────────────────────────────┐
│  calculating spatial position and  │
│  direction of the target by        │
│  comparing the three-dimensional   │          S140
│  coordinates of the markers with   │
│  the pre-stored geometric          │
│  information through the processor  │
└────────────────┬───────────────────┘
                 │
                 ▼
         ┌──────────┐
         │   End    │
         └──────────┘
```

**FIG. 5**

S130

```
        ┌─────────┐
        │  Start  │
        └─────────┘
             │
             ▼
┌──────────────────────────────────────┐
│   calculating two-dimensional         │
│   coordinates of images corresponding │─── S131
│   to the number of the lens           │
└──────────────────────────────────────┘
             │
             ▼
┌──────────────────────────────────────┐
│ performing calibration of the camera  │─── S132
│ for each coordinate                   │
└──────────────────────────────────────┘
             │
             ▼
┌──────────────────────────────────────┐
│  calculating three-dimensional        │
│  coordinates for each marker by using │
│  the 2-dimensional coordinates        │─── S133
│  of the images corresponding to the   │
│  number of the lens                   │
└──────────────────────────────────────┘
             │
             ▼
        ┌─────────┐
        │   End   │
        └─────────┘
```

**FIG. 6**

FOV of the first lens image    FOV of the second lens image

(U,V)                          (U',V')

131

**FIG. 7**

<table>
<tr><td colspan="2" align="center">**INTERNATIONAL SEARCH REPORT**</td><td colspan="2">International application No.<br><br>**PCT/KR2014/000426**</td></tr>
<tr><td colspan="4">A.    CLASSIFICATION OF SUBJECT MATTER<br>**_A61B 19/00(2006.01)i, A61B 1/313(2006.01)i_**<br>According to International Patent Classification (IPC) or to both national classification and IPC</td></tr>
<tr><td colspan="4">B.    FIELDS SEARCHED</td></tr>
<tr><td colspan="4">Minimum documentation searched (classification system followed by classification symbols)<br>A61B 19/00; A61B 5/05; G02B 5/12; G01M 19/00; G01B 11/00; A61B 1/04; B25J 19/00; A61B 1/313</td></tr>
<tr><td colspan="4">Documentation searched other than minimum documentation to the extent that such documents are included in the fields searched<br>Korean Utility models and applications for Utility models: IPC as above<br>Japanese Utility models and applications for Utility models: IPC as above</td></tr>
<tr><td colspan="4">Electronic data base consulted during the international search (name of data base and, where practicable, search terms used)<br>eKOMPASS (KIPO internal) & Keywords: surgical navigation, surgical navigation, marker, marker, lens, lens, tracking system, tracking system</td></tr>
</table>

C.    DOCUMENTS CONSIDERED TO BE RELEVANT

| Category* | Citation of document, with indication, where appropriate, of the relevant passages | Relevant to claim No. |
|---|---|---|
| A | JP 2007-130398 A (TOSHIBA CORP et al.) 31 May 2007<br>See figures 1-6, claims 1, 14, paragraphs [0018]-[0027] | 1-11 |
| A | KR 10-2011-0118640 A (INTUITIVE SURGICAL OPERATIONS, INC.) 31 October 2011<br>See claims 1, 13, paragraphs [0054]-[0067] | 1-11 |
| A | US 2007-0183041 A1 (MCCLOY, Bradley J. et al.) 09 August 2007<br>See abstract, paragraphs [0038]-[0039], claim 1 | 1-11 |
| A | US 2003-0209096 A1 (PANDEY, Rajesh et al.) 13 November 2003<br>See abstract, figures 2-3, paragraph [0020] | 1-11 |
| A | US 2005-0015005 A1 (KOCKRO, Ralf Alfons) 20 January 2005<br>See abstract, figures 16-18, claims 1, 5 | 1-11 |

| ☐   Further documents are listed in the continuation of Box C. | ☒   See patent family annex. |
|---|---|

<table>
<tr><td colspan="2">*   Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed</td><td>"T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family</td></tr>
<tr><td>Date of the actual completion of the international search<br><br>30 APRIL 2014 (30.04.2014)</td><td>Date of mailing of the international search report<br><br>**30 APRIL 2014 (30.04.2014)**</td></tr>
<tr><td>Name and mailing address of the ISA/KR<br>     Korean Intellectual Property Office<br>     Government Complex-Daejeon, 189 Seonsa-ro, Daejeon 302-701,<br>     Republic of Korea<br>Facsimile No.   82-42-472-7140</td><td>Authorized officer<br><br><br><br>Telephone No.</td></tr>
</table>

Form PCT/ISA/210 (second sheet) (July 2009)

INTERNATIONAL SEARCH REPORT
Information on patent family members

International application No.

**PCT/KR2014/000426**

| Patent document cited in search report | Publication date | Patent family member | Publication date |
|---|---|---|---|
| JP 2007-130398 A | 31/05/2007 | JP 4459155 B2 | 28/04/2010 |
| KR 10-2011-0118640 A | 31/10/2011 | CN 102341054 A | 01/02/2012 |
| | | EP 2391289 A1 | 07/12/2011 |
| | | US 2010-0168763 A1 | 01/07/2010 |
| | | WO 2010-078016 A1 | 08/07/2010 |
| US 2007-0183041 A1 | 09/08/2007 | CN 101379412 A | 04/03/2009 |
| | | CN 101379412 B | 10/08/2011 |
| | | DE 112007000340 T5 | 18/12/2008 |
| | | US 7945311 B2 | 17/05/2011 |
| | | WO 2007-090288 A1 | 16/08/2007 |
| US 2003-0209096 A1 | 13/11/2003 | CN 100491914C | 27/05/2009 |
| | | CN 1511249 A | 07/07/2004 |
| | | EP 1364183 A1 | 26/11/2003 |
| | | EP 1364183 A4 | 18/06/2008 |
| | | EP 1364183 B1 | 06/11/2013 |
| | | JP 2004-529679 A | 30/09/2004 |
| | | JP 2004-529679 T | 30/09/2004 |
| | | JP 4153305 B2 | 24/09/2008 |
| | | US 7043961 B2 | 16/05/2006 |
| | | WO 0206-1371A1 | 08/08/2002 |
| | | WO 0206-1371A1 | 08/08/2002 |
| US 2005-0015005 A1 | 20/01/2005 | CA 2523727 A1 | 06/01/2005 |
| | | EP 1617779 A1 | 25/01/2006 |
| | | JP 2007-512854 A | 24/05/2007 |
| | | JP 2007-512854 T | 24/05/2007 |
| | | US 7491198 B2 | 17/02/2009 |
| | | WO 2005-000139 A1 | 06/01/2005 |

Form PCT/ISA/210 (patent family annex) (July 2009)